# EUROPÄISCHE PATENTSCHRIFT

(11) **EP 0 242 835 B1**
(45) Veröffentlichungstag und Bekanntmachung des Hinweises auf die Patenterteilung: **03.02.1993**
(21) Anmeldenummer: 87105805.3
(22) Anmeldetag: 21.04.1987
(51) Int. Cl.: C12N 15/58, C12P 21/02

(54) **Verfahren zur Herstellung von Plasminogen-Aktivatoren in Prokaryonten**
Method for preparing plasminogen activators in procaryotes
Procédé de préparation des activateurs de plasminogène dans les procaryotes

(30) Priorität: 21.04.1986 DE 3613401
(43) Veröffentlichungstag der Anmeldung: 28.10.1987
(73) Patentinhaber: BOEHRINGER MANNHEIM GMBH, 68298 Mannheim (DE)
(72) Erfinder: Mattes, Ralf, Dr. rer. nat., D-8125 Oberhausen/Obb. (DE)
(74) Vertreter: Huber, Bernhard, Dipl.-Chem.

(56) Entgegenhaltungen:
- EP-A- 0 092 182
- EP-A- 0 093 619

## Beschreibung

Die Erfindung betrifft ein Verfahren zur Herstellung eines Plasminogenaktivators in einer Prokaryontenzelle.

Plasminogenaktivatoren sind in humanen und tierischen Geweben und Körperflüssigkeiten weit verbreitet. Sie spielen bei der Fibrinolyse eine zentrale Rolle und wandeln Plasminogen in Plasmin um. Plasmin ist wiederum für die Gerinnselauflösung von Bedeutung. Daher sind Plasminogenaktivatoren für die klinische Behandlung von Gefäßverschlüssen durch Blutgerinnsel von großem Interesse.

Bekannte Plasminogenaktivatoren sind beispielsweise Pro-Urokinase (u-PA) sowie tissue Plasminogenaktivator (t-PA).

Pro-Urokinase ist eine Serin-Protease in einkettiger Form, die durch Plasmin aktiviert wird (Eur. J. Biochem. 150 (1985), 183 - 188, EP-A1 0 092 182).

t-PA ist ebenfalls eine Serin-Protease, welche in verschiedenen Modifikationen vorkommt (Arteriosclerosis 4 (1984), 579 - 585).

Aus Gewebe oder Zellkulturen isolierter t-PA liegt in gering aktiver (zymogener) Form als einkettiger t-PA glycosiliert vor (Molekulargewicht ca. 70 000 Dalton). Durch limitierte Einwirkung von Plasmin, Trypsin oder Kallikrein auf das Zymogen, wird dieses in den vollaktiven Zustand (zweikettiger t-PA) übergeführt. Dabei wird die Bindung zwischen Arg 275 und Ile 276 gespalten. Es entsteht eine schwere Kette (A-Kette) sowie eine leichte Kette (B-Kette, Proteasendomäne). Beide Ketten bleiben über eine Disulfidbrücke verbunden (J. Biol. Chem. 256 (1981), 7035 - 7041).

Von t-PA sind außerdem sich geringfügig unterscheidende natürliche Derivate bekannt. So beginnen ca. 50 % der Moleküle mit Gly-Ala-Arg-Ser-Tyr-Gln (L-Kette), während bei den anderen ca. 50 % von t-PA Gly-Ala-Arg fehlt und die Aminosäure-Seqenz mit Gly/Ser-Tyr-Gln (S-Kette) beginnt. Schließlich kann noch an der Position L-4 bzw. S-1 ein Austausch von Gly/Ser erfolgen (FEBS Letters 156 (1983), 47 - 50). Diese geringfügigen Modifikationen haben jedoch keinen Einfluß auf die biologische Wirksamkeit von t-PA.

Da natürliche Plasminogenaktivatoren in der Natur nur in äußerst geringen Mengen (t-PA z.B. ca. 6 ng/ml in Plasma) vorkommen, werden die für die klinische Anwendung und wissenschaftliche Zwecke benötigten Mengen an diesen Aktivatoren vorteilhaft gentechnologisch hergestellt. Beispielsweise sind für t-PA sowohl Methoden für die Klonierung und Expression in Prokaryonten (Pennica et al, Nature 301 (1983), 214 - 221) als auch für die Expression in Eukaryonten (Biotechnology, Dezember 84, 1058 - 1062) beschrieben. Während die in Eukaryonten exprimierten Plaminogenaktivatoren glycosiliert sind, fehlt bei der Expression in Prokaryonten die Glycosilierung.

Es ist anzunehmen, daß die in Prokaryonten exprimierten Plasminogenaktivatoren biologisch ebenso wirksam sind, wie die glycosilierten natürlich vorkommenden Aktivatoren, weil beispielsweise bei t-PA nach Abspaltung der Kohlehydrat-Kette (Biochemistry 23 (1984), 6191 - 6195) oder Inhibierung der Glycosilierung (Thrombosis and Haemostasis 54 (1985), 788 - 791) ein so hergestellter nicht glycosilierter t-PA identische Eigenschaften mit dem glycosilierten natürlichen t-PA aufweist.

Auf Grund der unproblematischen Fermentation von Prokaryonten und der zu erwartenden hohen Ausbeuten besteht demzufolge ein großes Interesse an einem Verfahren zur Expression von Plasminogenaktivatoren in Prokaryonten. Die bisher bekannten Verfahren (vgl. für t-PA beispielsweise Nature 301 (1983), 214 -221 und EP OO 93 619) haben jedoch den Nachteil, daß überwiegend ein Protein exprimiert wird, welches nicht die gewünschte Kettenlänge hat. So wurde festgestellt, daß bei der Expression von t-PA c-DNA in E.coli mit pBR 322 als Vektor mehr als 80 % des exprimierten Proteins ein Molekulargewicht von nur 32 - 36 000 d aufweist und demzufolge aus Abbauprodukten von t-PA besteht. Weniger als 20 % des exprimierten Proteins ist einkettiger t-PA, dessen Molekulargewicht 57 000 d betragen sollte (entsprechend der in Nature, s.o., angegebenen Sequenz). Die Aufreinigung eines Gemischs von derart ähnlichen Verbindungen ist jedoch unwirtschaftlich und aufwendig.

Die beobachtete Bildung von t-PA-Abkömmlingen hat Ähnlichkeit mit einem seit langem bekannten Phänomen bei der Expression von rekombinanten Proteinen in Prokaryonten. Für verschiedene derartige Proteine wurde nämlich berichtet, daß das Protein zwar vollständig exprimiert werde, seine Halbwertszeit in der Wirtszelle aber nur gering sei, weil es einer Proteolyse unterliegt (Trends in Biotechnology, 1 (1983), 109 - 113). Um diesen proteolytischen Abbau zu vermeiden, wurden verschiedene Wege vorgeschlagen. Ein solcher Weg ist die Verwendung von Wirtszellen, bei denen das für die Spaltung verantwortliche proteolytische System fehlt.

Beispielsweise kann eine lon-Mutante von E.coli als Wirtszelle verwendet werden. Derartige Mutanten sind defizient bzgl. einer der im Wildtyp vorkommenden Protease. Da allerdings in E.coli mindestens sieben weitere Proteasen vorliegen (J. Bacteriol. 149 (1982), 1027 - 1033), wäre das Verfahren nur dann geeignet, wenn das zu exprimierende Protein von diesen anderen Proteasen nicht gespalten wird. Außerdem ist die Auswahl von geeigneten Wirtszellen sehr beschränkt.

Eine Proteinspaltung könnte auch dadurch vermieden werden, daß im Protein durch Aminosäurenaustausch (auf c-DNA-Ebene) die Proteasenschnittstelle entfernt wird. Dazu müßte allerdings diese Schnittstelle erst durch aufwendige Verfahren bestimmt werden. Außerdem kann der Aminosäurenaustausch die Aktivität und die immunologischen Eigenschaften des Proteins drastisch verändern.

Es ist weiter bekannt, in den Klonierungsvektor ein Antiproteasen-Gen des Phagen T₄ einzubauen (Proc. Natl. Acad. Sci. 80 (1983), 2059 -2062). Auch dieses Verfahren ist nur für bestimmte rekombinante Proteine geeignet und führt zudem nur zu einer unbefriedigenden Unterdrückung der Proteolyse.

Weiter wurde vorgeschlagen, die Expression durch die Erhöhung der Kopienzahl des Expressionsvektors soweit zu steigern, daß die Proteasen durch eine riesige Menge an rekombinantem Protein überschwemmt werden und demzufolge ihre Wirkung im Verhältnis nur bei einem geringen Prozentsatz des rekombinanten Proteins entfalten. Der Nachteil dieser Methode ist jedoch, daß die Expression innerhalb von nur 1 - 2 Generationen immens gesteigert werden muß und nicht längere Zeit durchgeführt werden kann (Trends in Biotechnology loc.cit.).

Ein weiteres Verfahren zur Vermeidung der Proteolyse besteht darin, daß ein rekombinantes Protein bereits auf c-DNA-Ebene durch Fusion mit einem weiteren Protein geschützt wird. Dieses Verfahren wurde beispielsweise für die Peptidhormone Insulin und Somatostatin beschrieben, wobei als Fusionspartner β-Galactosidase verwendet wird (Science 198 (1977), 1056 - 1063). Dieses Verfahren hat jedoch den Nachteil, daß das Schutzprotein mitexprimiert wird und bei der Aufreinigung erst wieder abgespalten und entfernt werden muß, was einen hohen zusätzlichen Aufwand bedeutet. Keines der bisher vorgeschlagenen Verfahren zur Verhinderung des Abbaus von gentechnologisch hergestellten Proteinen erscheint daher geeignet, das Problem bei der Bildung von Proteingemischen derart hergestellten Pa (Plasminogenaktivatoren) zu lösen.

Die Aufgabe der Erfindung besteht daher in der Bereitstellung eines Verfahrens, welches die Expression von vollständigen, im wesentlichen einheitlichen Plasminogenaktivatoren in Prokaryonten gestattet und die Nachteile der oben genannten Methoden zur Vermeidung einer Produktspaltung nicht aufweist.

Es wurde nun gefunden, und hierauf beruht die Erfindung, daß bei der Verwendung von Expressionsvektoren, deren Replikation von der Replikation des Chromosoms der Wirtszelle nicht oder nur vorübergehend entkoppelbar ist, so daß die Bildung von m-RNA wesentlich vermindert wird oder von Vektoren, deren Promotor so reguliert wird, daß die m-RNA-Bildung ebenfalls auf das verminderte Niveau herabgesetzt wird bei der Expression in Prokaryonten einkettiger Plasminogenaktivator in hoher Reinheit entsteht.

Das erfindungsgemäße Verfahren zur Herstellung eines Plasminogenaktivators durch Expression einer diesen Aktivator codierenden c-DNA, welche in einem für die Wirtszelle geeigneten Vektor eingebaut vorliegt, in einer Prokaryontenzelle, ist daher dadurch gekennzeichnet, daß man einen Vektor verwendet, der strikter Kontrolle unterliegt und dessen Replikation somit von der Replikation des Chromosoms der Wirtszelle nicht oder nur vorübergehend entkoppelbar ist und die Züchtung unter Bedingungen durchführt, bei denen keine relaxierte Kontrolle auftritt oder dessen Promotor derart reguliert wird, daß die m-RNA-Bildung bei entkoppelten Plasmiden nicht größer ist als bei den strikter Kontrolle unterliegenden Plasmiden.

Die Erfindung beruht auf der überraschenden Feststellung, daß die unerwünschte Bildung eines Proteingemisches auf das Auftreten einer Mischung von m-RNA-Ketten verschiedener Zusammensetzungen zurückzuführen ist und durch Einstellung einer entsprechend niedrigen Transkriptionsrate verhindert werden kann.

Vektoren, die strikter Kontrolle unterliegen, erhöhen in der frühstationären Phase des Wachstums der Wirtszelle ihre Kopienzahl um nicht mehr als den Faktor 10, vorzugsweise um nicht mehr als den Faktor 3. Sie werden als "low copy"-Plasmide bezeichnet, im Gegensatz zu den üblicherweise in der Gentechnologie verwendeten relaxiert kontrollierten Multicopy-Plasmiden.

Solche für das Verfahren der Erfindung geeignete Vektoren können gefunden werden, indem man überprüft, ob sie aktive Proteinbiosynthese oder eine DNA-Polymerase I für ihre Vermehrung benötigen.

Aus J. Bacteriol. 110 (1972), 667 -676) ist bekannt, daß durch Zusatz von Hemmstoffen der Protein-Biosysthese, wie Chloramphenicol oder Spectinomycin, die Plasmidreplikation von der Replikation der Chromosomen bei den relaxiert kontrollierten Vektoren (z.B. Vektoren des Col. E I-Typs, pBR 322) entkoppelt und die Kopienzahl der Vektoren beispielsweise auf mehr als 100 bis 1000 amplifiziert werden kann. Diese Eigenschaft begründet die bevorzugte Verwendung dieser Vektoren für Synthesezwecke wesentlich mit. Die für die erfindungsgemäße Verwendung geeigneten Vektoren, die strikter Kontrolle unterliegen, lassen sich durch diese Verfahren jedoch um nicht mehr als den Faktor 10, vorzugsweise um nicht mehr als Faktor 3 auf eine Kopienzahl von max. etwa 50 amplifizieren.

Vor Beginn des Wachstums liegen die Vektoren in einer Kopienzahl von 1 - 50 Kopien pro Zelle, vorzugsweise 2 bis 20, besonders bevorzugt 2 bis 10 Kopien vor. Sofern die Anfangskopienzahl des Vektors im unteren Bereich (unter oder gleich 10) liegt, sind auch solche Vektoren bevorzugt, deren Amplifikationsfaktor an der oberen Grenze des Bereichs (nahe 10) liegt. Sofern die Anfangskopienzahl bereits bei ca. 20 - 50 liegt, sollte der Amplifikationsfaktor vorteilhaft möglichst gering sein. Besonders geeignet sind dann Amplifikationsfaktoren gleich oder unter 3.

Als Vektoren geeignet sind die bevorzugten prokaryontischen Plasmidvektoren, Phagenvektoren und Kombinationen von beiden.

Bevorzugte Plasmidvektoren, die strikter Kontrolle unterliegen, sind pRSF 1010 (ein Derivat von pREM 3061 (DSM 3692 P), bei dem das Kanamycin-Resistenzgen deletiert wurde), pKN 402 (siehe z. B. Uhlin et al, Gene 6 (1979), 91 - 106) und pACYC 177 (DSM 3693 P) sowie hiervon abgeleitete Plasmide, welche vorteilhaft deren charakteristische Sequenz als Ori tragen. Besonders bevorzugt sind die Plasmide pKN 402 und pACYC 177. Die Replikation von pKN 402 läßt sich durch Temperaturerhöhung von der Replikation des Chromosoms entkoppeln. Dadurch kann die Kopienzahl auf einfache Weise erhöht werden. Hierbei zeigt sich dann aber ein drastischer Anstieg an nicht einkettigem Pa. Daher muß eine Temperatur, bei der keine Entkoppelung erfolgt, für die Züchtung gewählt werden. Tabellen 1 und 2 zeigen für einige Vektoren, die strikter oder relaxierter Kontrolle unterliegen, Kopienzahl und Bildung einkettiger Pa in % der durch diese Vektoren exprimierten Proteine.

Alternativ läßt sich die angestrebte niedrige Bildung von m-RNA-Abschriften des t-PA-Chromosoms bei Verwendung von Vektoren, die in hoher Kopienzahl vorliegen (high-copy-Vektoren) dadurch erreichen, daß man ihren Promotor so reguliert, daß die m-RNA-Bildung (Transkription) zu einer verringerten m-RNA-Bildung führt in gleicher Größenordnung wie bei den oben abgehandelten Vektoren, die strikter Kontrolle unterliegen. Bei Verwendung von derartigen high-copy-Vektoren mit starkem Promotor wird erfindungsgemäß der Promotor daher nur schwach induziert, so daß nur eine niedrige Transkription des t-PA-Gens in die m-RNA erfolgt. Beispielsweise lassen sich hierzu Wirtszellen verwenden, die einen Repressor bilden, der den Promotor des high-copy-Vektors entsprechend hemmt, wobei diese Hemmung aber durch Zugabe eines Induktors in entsprechend geringer Menge überwunden werden kann. Hierfür sind beispielsweise E.coli-Mutanten geeignet, die keine lac-Permease produzieren und sich so transformieren lassen, daß sie lac-Repressor überproduzieren. Verwendet man sie daher in Verbindung mit einem high-copy-Plasmid mit dem starken lac-Promotor, so wird dieser so stark reprimiert, daß die m-RNA-Bildung unterdrückt wird. Durch Zusatz eines Induktors kann dann eine angestrebte geringe m-RNA-Bildung erzielt werden. Ein typisches Beispiel für eine derartige Mutante ist der E.coli K12-Stamm, DSM 2102 oder DSM 2093. Wird dieser mit dem Plasmid pePa 119(DSM 3691P) transformiert, so handelt es sich um einen lac-Repressor-Überproduzenten. Er kann bei einem high-copy-Plasmid, dessen t-PA-Gen unter der Kontrolle des lac-Promotors liegt, zu der gewünschten m-RNA-Bildung verwendet werden, indem benötigte Induktoren in entsprechender Unterdosierung zugesetzt werden, beispielsweise IPTG (Isopropyl-β-D-thiogalactosid), oder bei einer lac-Permease Mangelmutante, Lactose. Im letzten Fall erfolgt die Unterdosierung des Induktors Lactose durch dessen stark verlangsamte Penetration in die Zelle.

Verwendet man Promotoren mit geringerer Effizienz als tac, kann bei voller Induktion eine entsprechend höhere Kopienzahl des Vektors erlaubt werden. Die Promoterstärke kann umgekehrt proportional der Kopienzahl gewählt werden oder die Nutzung des Promoters muß durch entsprechend niedrige Induktion eingeschränkt werden.

Der durch Limitierung der Kopienzahl des Expressionsvektors erreichbare Effekt, daß keine t-PA Bruchstücke produziert werden, kann auch auf folgende Weise erreicht werden:
Verwendet man einen high-copy-Expressionsvektor mit starkem Promoter (tac) in einem Stamm mit Lac-Repressorüberproduktion (lac I^{q}), so muß durch Zugabe von Induktor (IPTG, Lactose o.ä.) die t-PA-Synthese induziert werden. Dies gelingt mit Mengen von 0,5 bis 5 mM IPTG im Medium vollständig. Verwendet man hingegen low-copy-Vektoren im gleichen System (tac-Promoter, lac I^{q}) beobachtet man zwar die Bildung von refractile bodies aber keine Inkorporation von Bruchstücken des t-PA in den refractile bodies bei gleicher IPTG-Mengenzugabe.

Verringert man die IPTG-Zugabe bei high-copy-Vektoren auf 0,01 mM, so erhält man die gleichen reinen refractile bodies wie im low-copy-System bei voller IPTG-Induktion. Dieser Effekt wird mit schlechteren Promotern als tac analog erreicht. Die Kopienzahl des Vektors kann um den Wert gesteigert werden, um den der Promoter weniger effizient ist als der tac-Promoter. D.h. bei 1/10 Promotereffizienz mögliche Erhöhung der Plasmidkopienzahl um den Faktor 10 gegenüber den verwendeten low-copy Plasmiden.

Unter den beiden Varianten des erfindungsgemäßen Verfahrens wird die Verwendung des low-copy-Systems bevorzugt. Der Vorteil des erfindungsgemäßen low-copy-Systems mit starkem Promoter ist in der leichteren Handhabbarkeit des Systems in der Fermentation zu finden.Hier ist die Induktion weniger aufwendig durchführbar, da die Menge Induktor nicht durch Regelung scharf kontrolliert werden muß, sondern wie beschrieben, ein Eintopf-System funktioniert. Auch ist diese Induktion über das lac-System einer Steuerung über das trp-System vorzuziehen, da durch Lactose-Einsatz das Wachstum der Zellen mitgesteuert werden kann, was bei der trp-Verarmungstechnik (Genentech) weniger einfach möglich ist.

Das Molekulargewicht der Vektoren beträgt üblicherweise zwischen 10⁶ und 10⁸ Dalton. Die Vektoren können außer der Plasminogenaktivator -c-DNA, z.B. t-PA-c-DNA, vorteilhaft noch Regulations- und Terminationssequenzen sowie Selektionsmarker enthalten. Als Promotoren haben sich insbesondere der tac- und der trp-Promotor als geeignet erwiesen. Der Einbau der c-DNA in diese Vektoren kann in beliebiger Orientierung erfolgen.

**Tabelle I**

| Vektor | Kopienzahl | | % t-PA (einkettig) | Wirtszelle |
|---|---|---|---|---|
| | (Anfang) | (Frühstadium) | | |
| pRSF 1010 | 9 - 12 | 9 - 12 | > 90 % | E.coli/P. putida |
| pKN 402 30°C | 15 - 20 | < 50 | 90 % | E.coli |
| PACYC 177 | 10 - 20 | < 50 | 90 % | E.coli |
| pBR 322 | 30 - 60 | > 100 | 20 % | E.coli |
| pKN 402 40°C | 15 - 20 | > 500 | 10 % | E.coli |

**Tabelle II**

| Vektor | Kopienzahl | | % t-PA Derivat (Beispiel) | Wirtszelle |
|---|---|---|---|---|
| | (Anfang) | (Frühstadium) | | |
| pACYC 177 | 10 - 20 | < 50 | 90 % | E.coli |
| pRSF 1010 | 9 - 12 | 9 - 12 | > 90 % | E.coli und P. putida |
| pBR 322 | 30 - 60 | > 100 | 20 % | E.coli |

Als Wirtszellen sind Prokaryonten-Zellen geeignet. Vorteilhaft hat sich die Verwendung von E.coli, Klebsiella pneumoniae, Pseudomonas aeruginosa, Pseudomonas putida, und Bacillus subtilis erwiesen. Besonders bevorzugt wird von E.coli beispielsweise der Stamm DSM 3689, sowie von Pseudomonas putida der Stamm DSM 2106.

Die Wirtszellen werden zweckmäßigerweise je nach dem verwendeten Vektor so gewählt, daß letzterer im Wirt gut repliziert wird. Geeignete Vektor-Wirt-Kombinationen sind dem Fachmann bekannt. Zweckmäßig werden Wirtszellen verwendet, die einen für den jeweils verwendeten Promotor geeigneten Regulator (Repressor), z.B. lac-Repressor für den lac- oder tac-Promotor, enthalten.

Als Plasminogenaktivatoren werden im Rahmen der Erfindung alle eine Plasminogen aktivierende Wirksamkeit im oben definierten Sinne aufweisenden Proteine verstanden, vorzugsweise t-PA und Pro-Urokinase sowie deren Derivate.

Unter Derivaten werden sowohl die natürlichen Derivate verstanden als auch künstlich hergestellte Derivate, denen z.B. eine oder mehrere Domänenen teilweise bzw. ganz fehlen. Das Verfahren ist ebenso geeignet für Derivate, bei denen eine oder mehrere Aminosäuren ausgetauscht sind.

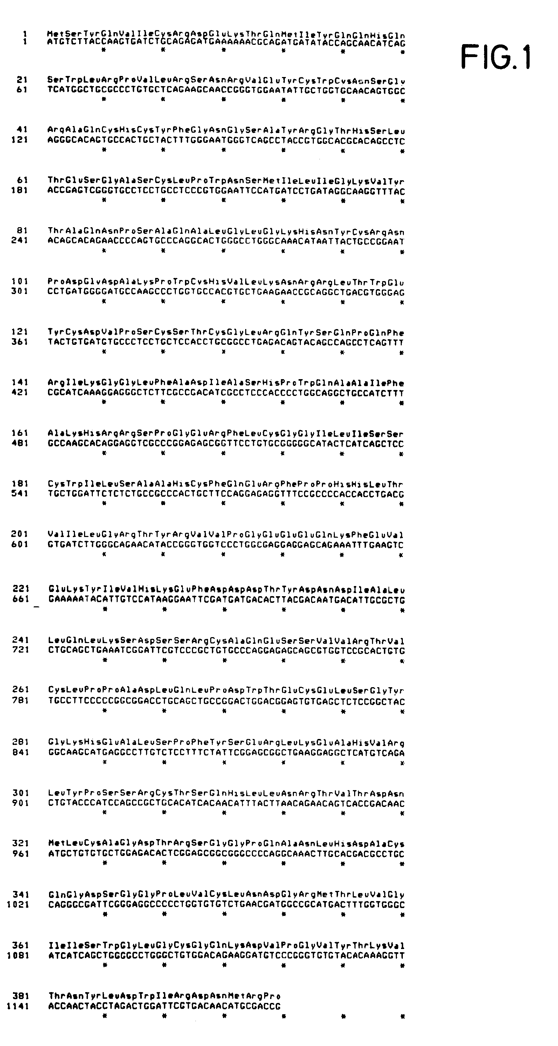

Besonders geeignet ist das Verfahren zur Expression von natürlichem t-PA sowie von t-PA-Derivaten, welche sich von dem bekannten natürlichen t-PA-Molekül ableiten (vgl. Figur 1), denen aber ein mit einer der Aminosäuren 43 bis 72 beginnendes und mit einer der Aminosäuren 179 bis 113 endendes Stück der Kette des vollständigen t-PA-Moleküls fehlt, wie sie in der deutschen Patentanmeldung P 36 43 158.3 beschrieben sind. Diese Nomenklatur ist analog der von Pennica loc.cit. beschriebenen Nomenklatur.

Die Einführung der den Plasminogenaktivator codierenden c-DNA in den Vektor erfolgt nach den dem Fachmann hierfür geläufigen Methoden, die hier nicht näher erläutert werden müssen. Soll beispielsweise ein tPA-Derivat hergestellt werden, dann verfährt man so, daß man aus der tPA-DNA denjenigen Abschnitt, der die dem Derivat im Vergleich zum kompletten tPA-Molekül fehlende Aminosäuresequenz kodiert, mit den entsprechenden Restriktionsendonukleasen herausschneidet, die das gewünschte tPA-Derivat kodierenden Abschnitte verbindet, die so erhaltene tPA-Derivat-cDNA über einen geeigneten Vektor gemäß der Erfindung in Prokaryonten einschleust und darin exprimiert. Als Restriktionsendonukleasen sind in diesem Falle insbesondere solche Enzyme geeignet, welche nur in dem Bereich von bp 315 bis bp 726 der cDNA-Sequenz (vgl. Pennica, Nature 301 (1983), 214 - 221) schneiden.

Besonders geeignet sind die Restriktionsendonukleasen-Kombinationen Dra III und Mae III zur Entfernung der Aminosäuren 45 - 179 oder Dde I und Mnl I zur Entfernung der Aminosäuren 45 - 171 sowie Dde I für die Aminosäuren 45 - 174 und Fnu 4 H zur Entfernung der Aminosäuren 52 - 168 und Rsa I zur Entfernung von den Aminosäuren 67 - 162.

Zur Wiederverknüpfung im Vektor werden die bekannten Methoden, vorzugsweise unter Verwendung von Linkern, angewendet.

Anschließend werden diese Vektoren in Prokaryonten nach den bekannten Verfahren eingeführt und die Plasminogenaktivatoren wie z.B. tPA-Derivate exprimiert.

Ebenso kann zur Herstellung der fertigen Vektoren von der vollständigen DNA, welche Introns enthält, ausgegangen werden. Hierzu wird die DNA, z.B. tPA-DNA aus der Maniatis-Genbank, isoliert, in den ausgewählten Vektor eingesetzt.

Ferner kann aus PA-produzierenden Zellinien (Literatur Pennica, loc.cit.) die m-RNA isoliert werden und durch Größenfraktionierung aufgetrennt werden. Durch c-DNA-Herstellung daraus und Untersuchung der entstandenen Klone können die Klone gefunden werden, welche die codierende Region für PA enthalten. Dazu werden Klone ausgewählt, die mit entsprechend konstruierten Oligonukleotiden hybridisieren.

Besonders bevorzugt wird erfindungsgemäß ein tPA-Derivat hergestellt, bei dem die Aminosäuren 45 - 179 fehlen. Dieses kann beispielsweise auf cDNA-Ebene hergestellt werden, durch Schnitt mit den Restriktionsendonukleasen Dra III und Mae III und Verdau der überstehenden Einzelstrangenden mit Nuclease S1. Anschließend wird mit Ligase ligiert. Dann wird mit einem erfindungsgemäßen Vektorsystem in Prokaryonten exprimiert. Das bei der Expression in Eukaryonten so entstandene tPA-Derivat hat ein Molekulargewicht von ca. 43 000 D.

Erfindungsgemäß erhält man praktisch reinen Plasminogenaktivator hauptsächlich in ungelöster inaktiver Form (refractile bodies), der sich nach Abtrennung in lösliche, aktive Form überführen läßt.

### Beispiel 1

### Konstruktion der Expressionsplasmide für tPA:

### a) Konstruktion eines Expressionsplasmides unter Verwendung eines Plasmides mit dem pBR 322 ori:

Als Ausgangsplasmid dient das Plasmid pBT 95 (DSM 3611 P, DE 35 45 126), aus dem das Plasmid pePa 98.1 auf folgende Weise hergestellt wurde: Das Plasmid wird mit dem Enzym Bgl II gespalten und mit der Nuclease Sl nachbehandelt. Durch weitere Spaltung mit dem Enzym Sca I läßt sich ein Fragment a präparativ erhalten, das ca. 760 Basenpaare groß ist und die für tPA kodierende Nucleotid-Sequenz 192-952 (Pennica loc.cit.) enthält. Ein Fragment b wird ebenfalls aus pBT 95 erhalten durch Spaltung mit Sca I und Hind III; dadurch erhält man ein Fragment von etwa 1200 Basenpaaren, das die tPA-Nucleotid-Sequenz 953-2165 enthält. Ein Partner c wird als Linker synthetisch hergestellt und enthält die folgende Sequenz: 
5'GAATTCTTATGTC3'
3'     GAATACAG5'
Als Partner d in der Konstruktion wird das Plasmid pKK 223-3 (DSM 3694 P) mit den Enzymen Eco RI und Hind III im Polylinker gespalten. Die Partner a-d werden zusammen ligiert. Der Ligationsansatz wird nach üblicher Technik mit T4-Ligase behandelt und anschließend in E.coli-Zellen (DSM 3689) transformiert. Die transformierten Zellen werden auf Medium unter Zusatz von 50 µg/ml Ampicillin kultiviert. Aus den erhaltenen Klonen werden diejenigen ausgewählt, die das Plasmid pePa 98.1 tragen, das sich von den Ausgangsplasmiden pBT 95 und pKK 223-3 dadurch unterscheidet, daß es im Polylinker des Plasmides pKK 223-3 zwischen der Eco RI und Hind III-Spaltstelle die tPA-Nucleotid-Sequenz 192-2165 in richtiger Reihenfolge enthält.

### b) Konstruktion eines Expressionsplasmides für tPA mit Temperatur-sensitiver Replikationssteuerung:

Aus dem in 1a) hergestellten Plasmid pePa 98.1 kann durch Spaltung mit Xho II ein etwa 1,85 kb großes Fragment gewonnen werden, das für tPA kodiert und den tac-Promotor enthält. In ihm ist die für tPA kodierende Nucleotid-Sequenz 192-1809 unverändert enthalten. Durch Behandlung mit dem Klenow-Enzym in Gegenwart von allen 4 Desoxynucleosidtriphosphaten werden die 5'-überhängenden Enden der Xho II-Spaltstellen aufgefüllt. Das Empfängerplasmid pREM 2334 (DSM 3690 P), dessen Replikation und damit die Kopienzahl durch Temperatursteuerung beeinflußt werden kann, wird mit dem Enzym Cla I linearisiert und die 5'-überstehenden Enden werden mit dem Klenow-Enzym in Gegenwart von allen 4 Desoxynucleosidtriphosphaten aufgefüllt. Die so vorbereiteten Partnermoleküle werden in einem Ligierungsansatz vereinigt. Nach Transformation in E.coli-Zellen (DSM 3689) werden die Zellen auf Nährmedium mit 25 µg/ml Chloramphenicol gezüchtet. Die Temperatur für die Bebrütung darf nur 30°C betragen. Von den so erhaltenen Klonen werden diejenigen ausgewählt, die das Plasmid pePa 100.1 enthalten. Dieses unterscheidet sich vom Ausgangsplasmid pREM 2334 darin, daß es das Xho II-Fragment, das für tPA kodiert, enthält. Dieses wird überprüft, indem man die Plasmide dieser Klone isoliert und mit Bam HI spaltet. pePa 100.1-Moleküle lassen sich so in 2 Fragmente zerlegen, die ca. 6,85 und 2,15 kb groß sind.

### c) Konstruktion eines Expressionsvektors für tPA, der sowohl in E.coli wie in Pseudomonas putida verwendet werden kann.

Das im Beispiel 1b) beschriebene Xho II-Fragment, das mit Klenow-Enzym behandelt worden war, wird wiederum verwendet. Als Empfängerplasmid dient das Plasmid pREM 3061 (DSM 3692 P), das mit Hpa I linearisiert wird. Beide Partnermoleküle werden gemeinsam in einem Ligationsansatz mit T4-Ligase behandelt. Nach Transformation in E.coli-Zellen (DSM 3689) und Züchtung der erfolgreich transformierten Zellen auf Nährmedium mit 25 µg/ml Kanamycin werden Klone erhalten. Aus diesen werden diejenigen Zellen ausgewählt, die das Plasmid pePa 107.8 enthalten. Dieses unterscheidet sich vom Ausgangsvektor pREM 3061 dadurch, daß es das beschriebene Xho II-Fragment enthält. Das Plasmid wird aus den Zellen gewonnen und mit dem Enzym Bst E II analytisch gespalten. Man erhält dabei 2 Fragmente der ungefähren Größe von 10,8 und 1,3 kb. Dieses Plasmid kann durch Transformation in Zellen des Stammes Pseudomonas putida eingeführt werden.

Dazu werden Zellen des Stammes Pseudomonas putida (DSM 2106) zuerst mit dem Plasmid pePa 119 (DSM 3691 P) transformiert und auf Nährmedium mit 25 µg/ml Kanamycin selektioniert. Diese Pseudomonas putida-Zellen enthalten nun ein Plasmid, das in der Lage ist, lac-Repressor in großer Menge zu produzieren.

Die pePa 119 enthaltenden Pseudomonas putida-Zellen werden mit dem Plasmid pePa 107.8 transformiert. Die Transformanten werden auf Nährmedium mit 50 µg/ml Streptomycin selektioniert und enthalten danach sowohl die Plasmide pePa 119 wie pePa 107.8. Die Anwesenheit des Plasmides pePa 119 unterdrückt die Produktion von tPA in den Zellen durch Produktion des lac-Repressorproteins. Dieses kann die Transkription vom tac-Promotor aus auch im Ps. putida reprimieren.

### d) Konstruktion eines Expressionsplasmides für tPA mit dem ori von pACYC 177.

Verwendet wird wiederum das ca. 1,85 kb große Xho II-Fragment, das mit Klenow-Enzym behandelt wurde (aus Beispiel 1b). Dieses wird Fragment a genannt. Ein Fragment b wird aus pKK 223-3 präpariert und zwar durch Spaltung mit Bam HI und Behandlung mit Sl-Nuclease. Anschließend wird mit dem Enzym Pvu I nachgespalten, man erhält so ein ca. 1,05 kb großes Fragment, das Transkriptionsterminatoren enthält und die 5'-Portion des β-Lactamasegens. Ein Fragment c erhält man durch Spaltung des Plasmides pACYC 177 (DSM 3693 P) mit Bam HI und anschließendem Sl-Nuclease-Verdau. Dieser Ansatz wird dann partiell mit Pvu I nachgespalten. Präparativ kann ein ca. 2,9 kb großes Fragment erhalten werden. Die Fragmente a, b und c werden in einem Ligationsansatz vereinigt. Nach Behandlung mit T4-Ligase wird dieser Ligationsansatz transformiert in Zellen von E.coli (DSM 3689). Die Zellen werden anschließend auf Nährmedium mit 25 µg/ml Kanamycin und 50 µg/ml Ampicillin plattiert. Von den entstandenen Klonen werden diejenigen ausgewählt, die das Plasmid pePa 133 tragen. Dieses unterscheidet sich von dem Ausgangsplasmid pACYC 177 dadurch, daß es die tPA kodierende Sequenz von Position 192-1809 unverändert enthält und zusätzlich die Transkriptionsterminatoren aus dem Plasmid pKK 223-3. Das Plasmid wird isoliert und mit dem Enzym Bst E II analytisch gespalten. Man erhält zwei Fragmente der Größe von ca. 3,9 und 1,9 kb.

### Beispiel 2

### Expression des tPA-Proteins in Prokaryontenzellen

a) In 1 a), b), c) und d) hergestellte Plasmide werden E.coli (DSM 3689) transformiert, der ein lac I^{q}-Plasmid enthält. Die Transformanten werden auf Medien mit 50 µg/ml Ampicillin für die Plasmide aus 1 a), c) und d) bzw. 25 µg/ml Chloramphenicol für das Plasmid aus 1 b) selektioniert. Die Zellen, die also die Plasmide pePa 98.1, pePa 100.1, pePa 107.8, oder pePa 133 enthalten, werden in Nährboullion bis zu OD ₅₅₀ₙₘ = 0,4 unter Belüftung gezüchtet und dann unter Zusatz von 100mM IPTG induziert. Die Bebrütung geschieht bei 37°C für die Plasmide pePa 98.1, pePa 107.8 und pePa 133, jedoch 30°C für das Plasmid pePa 100.1. Nach 4 Stunden Bebrütung unter Belüftung in Gegenwart von IPDG werden die Zellen geerntet und aufgeschlossen. Dazu werden sie für 15 min auf Eis mit 0,5 µg/ml Lysozym behandelt (Zellkonzentration 10 OD/ml). Verwendet wird ein Tris-Puffer, pH 8,6 (0,1 M/l Tris-HCl mit 100 mM/l EDTA und 100 mM/l NaCl). Danach werden die Zellen durch Ultraschallbehandlung aufgeschlossen. Die so erhaltene Suspension wird für 10 min bei 20.000 g zentrifugiert und der Überstand verworfen. Das Sediment enthält das tPA-Protein in inaktiver Form. Dieses kann nach der in DE 35 37 708 beschriebenen Methode gelöst und reaktiviert werden. Das gewonnene Pellet kann aber auch durch Zusatz von 1 % SDS und 100 mM/l Mercaptoäthanol gelöst werden und in einem SDS-Polyacrylamidgel elektrophoretisch aufgetrennt werden. Durch Einfärbung der Proteine nach erfolgter Elektrophorese mittels Choomassieblue können die Proteinbanden sichtbar gemacht werden. Der Extrakt aus den Zellen, die die Plasmide pePa 100.1, pePa 107.8 oder pePa 133 tragen, enthält hauptsächlich 1 Protein des Molekulargewichts von ca. 57.000 Dalton, das der Größe des unglykosylierten tPA-Proteins entspricht. Der Extrakt aus den Zellen, die das Plasmid pePa 98.1 tragen, enthält hauptsächlich Material der Größenordnung 32-34.000 Dalton und nur eine geringe Menge der Größe 57.000 Dalton. Die identifizierten Proteinbanden können nach dem Western-blot-Verfahren mit Anti-tPA-PAK-Konjugat aus Ziege gleichwertig immunologisch nachgewiesen werden.
b) Pseudomonas pudita-Zellen (DSM 2106), die mit den Plasmiden pePa 119 und pePa 107.8 gemäß Beispiel 1c) transformiert wurden, werden bei 30°C in Nährboullion bis zu oD ₅₅₀ₙₘ = 0,4 unter Belüftung gezüchtet und dann unter Zusatz von 10 mM IPTG induziert. Nach 4 Stunden Bebrütung bei 30°C unter Belüftung in Gegenwart von IPTG werden die Zellen geerntet und aufgeschlossen. Im weiteren wird wie unter 2a) beschrieben verfahren. Nach erfolgter Gelelektrophorese des Zellextraktes und Anfärbung der Proteine durch Coomassie-blue wird im Extrakt hauptsächlich ein Protein des Molekulargewichts von ca. 57.000 Dalton gefunden. Dieses Protein ist als tPA-Protein nach dem Western-blot-Verfahren mit Anti-tPA-PAK-Konjugat aus Ziege immunologisch nachweisbar. Proteine, die kleiner sind als 57.000 Dalton, sind in der Regel immunologisch auf dem beschriebenen Wege nicht als tPA nachweisbar.

### Beispiel 3

### Vergleich der tPA-Expression mit hoher und niedriger Kopienzahl des Vektorplasmides:

Verwendet wird ein E.coli-Stamm (DSM 3689), der das Plasmid pePa 100.1 enthält. Die Zellen werden in Nährboullion bis zu oD ₅₅₀ₙₘ = 0,4 unter Belüftung bei 30°C gezüchtet. Durch Zugabe von 10 mM IPTG werden sie anschließend induziert. Der Ansatz wird in 3 gleichgroße Volumenteile geteilt. Jeweils ein Drittel wird weiter bei 30°C oder bei 37°C oder bei 42°C für 4 Stunden in Gegenwart von IPTG weiter inkubiert. Die Zellen werden anschließend geerntet und wie unter Beispiel 2 beschrieben, aufgeschlossen. Nach SDS-Gelelektrophorese der Extrakte können die tPA-Proteinbanden mit Coomassie-blue sichtbar gemacht werden. Der Extrakt aus dem 30°C-Ansatz enthält hauptsächlich ein Protein des Molekulargewichts 57.000 Dalton. Die Extrakte aus den Ansätzen bei 37°C und 42°C enthalten nur einen geringen Anteil des Proteins der Größe 57.000 Dalton, aber einen sehr großen Anteil der Größenordnung 32.000 bis 34.000 Dalton. Diese Proteinbanden lassen sich nach den Westernblot-Verfahren mit Anti-tPA-PAK-Konjugat aus Ziege gleichwertig immunologisch nachweisen.

Das Plasmid pePa 100.1 wird bei höheren Temperaturen stärker repliziert, was zu einer höheren Kopienzahl des Plasmides in den Zellen führt. Diese höhere Kopienzahl wirkt sich nachteilig auf den Erhalt von inaktivem tPA-Protein der Größenordnung 57.000 Dalton aus.

### Beispiel 4

Renaturierung von tPA-Proteinen aus Prokaryonten.

Die gemäß Beispiel 2 und 3 gewonnenen Zellfraktionen, die tPA-Protein enthalten können nach der in DE 35 37 708 beschriebenen Methode gelöst und reaktiviert werden. Dabei wird aus Extrakten der Zellen die pePa 107.8 oder pePa 133 oder pePa 100.1 gezüchtet bei 30°C etwa 10mal mehr aktives tPA erhalten, als aus Zellen, die pePa 98.1 oder aber pePa 100.1 gezüchtet bei 37°C oder 42°C enthalten. Das gewonnene aktive tPA ist jeweils auch durch Fibrin stimulierbar. Die Stimulierbarkeit ist in der Regel größer als 10fach.

### Beispiel 5

### Deletion der die Halbwertzeit bestimmenden Domänen von tPA

### a) Konstruktion des tPA-Mutein-Gens

Als Ausgangsmaterial wird das Plasmid pePa 98.1 verwendet. Aus diesem Plasmid wurde das tPA-codierende Fragment mit dem tac-Promotor über die Xho II-Spaltung erhalten. Dieses Fragment ist etwa 1,85 kb groß und wird an den überstehenden Enden durch Behandlung mit Klenow-Enzym, in Gegenwart von allen vier Desoxinukleosidtriphosphaten vollständig aufgefüllt. In einem Schritt A wird dieses Fragment mit dem Enzym Dra III geschnitten und die überstehenden Enden mit der Nuclease Sl abgedaut. Gelelektrophoretisch wird das Fragment der Größe von ca. 370 Basenpaaren gewonnen. In einem Schritt B wird das gleiche Xho II Ausgangsfragment mit dem Enzym Mae III geschnitten und ebenfalls mit Sl nachverdaut. Anschließend wird dieser Ansatz zusätzlich mit dem Enzym Sac I behandelt. Gelelektrophoretisch wird daraus ein Fragment der Größe von ca. 700 Basenpaaren isoliert. In einem Ansatz C wird das Vektorplasmid pePa 98.1 mit dem Enzym BamH I geschnitten und die überstehenden Enden mit dem Klenow-Enzym, unter Einsatz von allen vier Desoxinukleosidtriphosphaten aufgefüllt und mit dem Enzym Sac I nachgespalten. Gelelektrophoretisch wird anschließend das größte Fragment aus diesem Ansatz gewonnen. In einem Ligierungsansatz werden die gewonnenen Fragmente aus den Ansätzen A, B und C vereinigt und unter Zusatz von DNA-Ligase ligiert.

Die Ligierungsmischung wird in E.coli-DSM 3689, welche ein lac I^{q}-Plasmid enthalten, transformiert und die entstehenden Transformanten werden auf Nährmedium mit 50µg/ml Ampicillin selektioniert. Aus den so erhaltenen Klonen werden diejenigen ausgewählt, die das gewünschte Plasmid pePa 129 enthalten, das sich vom Ausgangsplasmid pePa 98.1 dadurch unterscheidet, daß es die DNA-Sequenz zwischen der tPA cDNA-Sequenz Pos. 301 bis 726 nicht mehr enthält. Figur 1 zeigt die Nucleotidsequenz des so erhaltenen Muteingens.

### b) Konstruktion eines Expressionsvektors für das Mutein zur Expression in E. coli

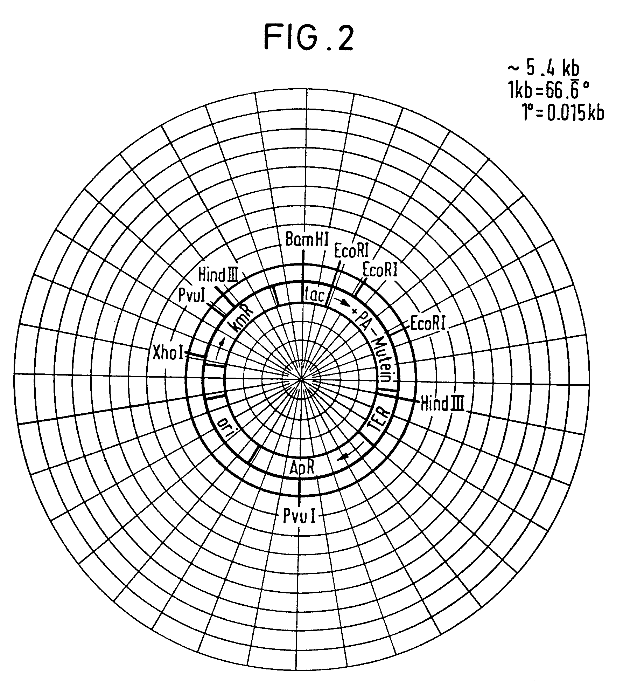

Aus dem nach 5 a) hergestellten Plasmid pePa 129 wird durch Verdau mit den Restriktionsenzymen Bam H I und Pvu I das tPA-codierende Fragment erhalten. Das Plasmid pACYC 177 (DSM 3693 P) wird ebenfalls mit Bam H I und limitiert mit Pvu gespalten. Beide Fragmente werden zusammenligiert und in E.coli-DSM 3689, welche ein lac I^{q}-Plasmid enthalten, transformiert. Durch Selektion auf Kanamycin und Ampicillin mit je 25 bzw. 50 µg/ml erhält man Transformanten, unter denen diejenigen ausgewählt werden, die das Plasmid pePa 137 enthalten. Diese unterscheidet sich von den Ausgangsvektoren dadurch, daß es das Mutein-Genfragment aus pePa 129 und die Sequenz des Plasmids pACYC 177 jeweils vollständig enthält. Eine Restriktionsschnittstellenkarte des Plasmids pePa 137 zeigt Figur 2.

### c) Konstruktion eines Expressionsvektors für das Mutein zur Expression in Pseudomonas putida

Als Vektor für Pseudomonas putida wird ein Derivat des Plasmides pRSF 1010 verwendet, welches zusätzlich ein Kanamycin-Resistenzgen aus pACYC 177 enthält. Dieser Vektor führt die Bezeichnung pREM 3061 (DSM 3692 P). Dieses Plasmid wird durch Verwendung des Restriktionsenzyms Hpa I linearisiert. Aus dem nach 5 a) hergestellten Vektor pePa 129 wird durch Xho II-Spaltung ein ca. 1,45 kb großes Fragment gewonnen, das den tac-Promotor und die vollständige Mutein-Gensequenz enthält. Durch Behandlung mit Klenow-Fragment, in Gegenwart von allen vier Desoxynukleosidtriphosphaten werden die Xho II-Schnittstellen vollständig aufgefüllt. Das so behandelte Fragment wird mit dem linearisierten Vektor pREM 3061 ligiert und in E.coli-Zellen (DSM 3689), transformiert. Die Transformanten werden auf Medium mit 25 µg/ml Kanamycin selektioniert. Transformanten, die das Plasmid pePa 143 enthalten, werden ausgewählt. Dieses Plasmid unterscheidet sich vom Ausgangsvektor pREM 3061 dadurch, daß es die vollständige Mutein-Gensequenz mit tac-Promotor enthält. Dieses Plasmid wird isoliert und durch Transformation in Zellen des Stammes Pseudomonas putida, DSM 2106 eingeführt. Die Transformanten werden auf Medium mit 25 µg/ml Kanamycin selektioniert und anschließend analysiert. Sie enthalten das Plasmid pePa 143 Zusätzlich kann in diese Transformanten-Zellen ebenfalls durch Transformation ein Plasmid pePa 119 (DSM 3691 P) eingeführt werden, das mit pePa 143 kompatibel ist und das lac I^{q}-Gen enthält. Dieses Plasmid ist ein Derivat vom Plasmid RP 4. Die Anwesenheit dieses Plasmides unterdrückt die Produktion des Muteins in den Zellen durch Produktion des lac-Repressor-Proteins. Dieses kann die Transkription vom tac-Promotor aus reprimieren.

### Beispiel 6

### Expression von intaktem t-PA in E.coli mit high-copy-Vektoren mit starkem Promoter

Verwendet wird ein Derivat von E.coli K-12, der eine Mutation im lac-I^{q}-Gen besitzt und die lac-Permease nicht produzieren kann. Der Stamm ist ED 8654 (DSM 2102) oder C600 (DSM 2093). Von diesem Stamm können Varianten hergestellt werden, die lac-Repressor überproduzieren (siehe 1a) indem sie mit pePa 119 (DSM 3691P) transformiert werden. Transformiert man zudem in diese so erhaltenen Bakterien das Plasmid pePa 98.1 (siehe 1a) erhält man Bakterien, die lac-Repressor überproduzieren und hier t-PA synthetisieren.

Man züchtet diese Bakterien wie unter 2a beschrieben in Nährboullion bei 37°C unter Belüftung.
a) Bei Erreichen von Od₅₅₀=0,4 wird 0,2 % Lactose zum Medium hinzugefügt und die Bebrütung für 4 Stunden fortgesetzt. Durch das Fehlen von lac-Permease erfolgt nur eine langsame Aufnahme der Lactose in die Zellen. Wie unter Beispiel 2a beschrieben, werden anschließend die Zellen geerntet, aufgeschlossen, die t-PA-Fraktion gewonnen und im SDS-Polyacrylamidgel analysiert.
   Man erhält qualitativ und quantitativ die gleichen Ergebnisse für die t-PA-Produktion wie für Extrakte aus vollinduzierten low-copy-Vektoren aus Beispiel 2.
b) Desgleichen kann durch Zusatz von geringen Mengen IPTG zu diesem Zeitpunkt eine entsprechend geringe Induktionsrate für t-PA-Expression erreicht werden. Mit Mengen von 0,05 bis 5 mM IPTG und darüber gelingt eine vollständige Induktion, während mit Mengen zwischen 0,002 und 0,01 mM eine entsprechend niedrigere Induktion erreicht wird. Diese geringe Induktion führt zum qualitativ und quantitativ gleichen Ergebnis wie die volle Induktion von low-copy-Vektoren aus Beispiel 2.
c) Verwendet man Promotoren mit geringerer Effizienz als tac, kann bei voller Induktion eine entsprechend höhere Kopienzahl des Vektors erlaubt werden. Die Promoterstärke kann umgekehrt proportional der Kopienzahl gewählt werden oder die Nutzung des Promoters muß durch entsprechend niedrige Induktion eingeschränkt werden.

Der durch Limitierung der Kopienzahl des Expressionsvektors erreichbare Effekt, daß keine t-PA Bruchstücke produziert werden, kann auch auf folgende Weise erreicht werden:

Verwendet man einen high-copy-Expressionsvektor mit starkem Promoter (tac) in einem Stamm mit Lac-Repressorüberproduktion (lac I^{q}), so muß durch Zugabe von Induktor (IPTG, Lactose o.ä.) die t-PA-Synthese induziert werden. Dies gelingt mit Mengen von 0,5 bis 5 mM IPTG im Medium vollständig. Verwendet man hingegen low-copy-Vektoren im gleichen System (tac-Promoter, lac I^{q}) beobachtet man zwar die Bildung von refractile bodies aber keine Inkorporation von Bruchstücken des t-PA in den refractile bodies bei gleicher IPTG-Mengenzugabe.

Verringert man die IPTG-Zugabe bei high-copy-Vektoren auf 0,01 mM erhält man die gleichen reinen refractile bodies wie im low-copy-System bei voller IPTG-Induktion. Dieser Effekt sollte mit schlechteren Promotern als tac analog erreicht werden. Die Kopienzahl des Vektors kann um den Wert gesteigert werden, um den der Promoter weniger effizient ist als der tac-Promoter. D.h. bei 1/10 Promotereffizienz mögliche Erhöhung der Plasmidkopienzahl um den Faktor 10 gegenüber den verwendeten low-copy Plasmiden.

Der Vorteil des erfindungsgemäßen low-copy-Systems mit starkem Promoter ist in der leichteren Handhabbarkeit des Systems in der Fermentation zu finden.Hier ist die Induktion weniger aufwendig durchführbar, da die Menge Induktor nicht durch Regelung scharf kontrolliert werden muß, sondern wie beschrieben, ein Eintopf-System funktioniert. Auch ist diese Induktion über das lac-System einer Steuerung über das trp-System vorzuziehen, da durch Lactose-Einsatz das Wachstum der Zellen mitgesteuert werden kann, was bei der trp-Verarmungstechnik (Genentech) weniger einfach möglich ist.

## Patentansprüche (Patentansprüche für folgende(n) Vertragsstaat(en): BE, CH, DE, ES, FR, GB, GR, IT, LI, LU, NL, SE)

1. Verfahren zur Herstellung eines Plasminogenaktivators durch Expression einer diesen Aktivator codierenden c-DNA, welche in einem für die Wirtszelle geeigneten Vektore eingebaut vorliegt, in einer Prokaryontenzelle,
**dadurch gekennzeichnet,**
daß man einen Vektor verwendet, der entweder strikter Kontrolle unterliegt und dessen Replikation somit von der Replikation des Chromosoms der Wirtszelle nicht oder nur vorübergehend entkoppelbar ist und die Züchtung unter Bedingungen durchführt, bei denen keine relaxierte Kontrolle auftritt, oder einen Promotor verwendet, unter dessen Regulation auch bei entkoppelten Plasmiden nur eine geringe Transkription des Plasminogen-Aktivatorgens erfolgt.

2. Verfahren nach Anspruch 1, dadurch gekennzeichnet, daß man einen Vektor verwendet, dessen Amplifikationsfaktor nicht größer als 3 ist.

3. Verfahren nach Anspruch 1 oder 2, dadurch gekennzeichnet, daß man als Vektor pRSF 1010 (ein Derivat von pREM 3061 (DSM 3692P), bei dem das Kanamycin-Resistenzgen deletient wurde), pKN 402, pACYC 177 (DSM 3693P) oder davon abgeleitete Plasmide, die deren Ori tragen, verwendet.

4. Verfahren nach Anspruch 3, dadurch gekennzeichnet, daß man die c-DNA eines Plasminogenaktivators in pKN 402 verpackt, in eine für diesen Vektor geeignete Prokaryonten-Wirtszelle einführt und die Wirtszelle bei einer Temperatur um 30°C züchtet.

5. Verfahren nach Anspruch 3 oder 4, dadurch gekennzeichnet, daß man als Wirtszelle E. coli DSM 3689 verwendet.

6. Verfahren nach Anspruch 3, dadurch gekennzeichnet, daß man bei pRSF 1010 als Wirtszelle Pseudomonas putida DSM 2106 verwendet.

7. Verfahren nach Anspruch 5,
**dadurch gekennzeichnet,**
daß man E.coli DSM 3689 züchtet, welches Plasmid pePa 100.1 oder/und pePa 107.8 oder/und pePa 133 oder pePa 137 enthält,
wobei das Plasmid pePa 100.1 ein tPA-Expressionsvektor mit temperatursensitiver Replikationssteuerung ist, der durch Ligation des mit einem temperatursensitiven Replikationsursprung versehenen Plasmids pREM 2334 (DSM 3690 P) mit einem 1,85 kb langen, XhoII-Fragment aus pePa 98.1, das für tPA kodiert und den tac-Promotor enthält, gewonnen wird, wobei pePa 98.1 die tPA-Nucleotidsequenz 192-2165 (Pennica) aus dem Plasmid pBT95 (DSM 3611 P) im Polylinker der Plasmids pKK223-3 (DSM 3694 P) enthält, das Plasmid pePa 107.8 ein zur Expression in E.coli und Pseudomonas putida geeigneter tPA-Expressionsvektor ist, der durch Ligation des Plasmids pREM 3061 (DSM 3692 P) mit dem oben beschriebenen 1,85 kb langen XhoII-Fragment aus pePa 98.1, das für tPA kodiert und den tac-Promotor enthält, gewonnen wird,
das Plasmid pePa 133 ein tPA-Expressionsvektor ist, der durch Ligation eines ca. 1,05 kb großen Fragments aus pKK223-3 (DSM 3694 P), das Transkriptionsterminatoren und die 5'-Portion des β-Lactamasegens enthält, mit einem ca. 2,0 kb großen Fragment aus dem Plasmid pACYC177 (DSM 3693 P), das den Replikationsursprung enthält, und dem oben beschriebenen 1,85 kb langen XhoII-Fragment aus pePa 98.1, das für tPA kodiert und den tac-Promotor enthält, gewonnen wird,
und das Plasmid pePa 137 ein tPA-Mutein-Expressionsvektor ist, der durch Ligation eines Fragments aus pACYC 177 (DSM 3693 P), das den Replikationsursprung enthält, mit einem Fragment aus pePa 129, das die tPA-Mutein-Nucleotidsequenz gemäß Fig. 1 enthält, gewonnen wird, wobei pePa 129.1 aus pePa 98.1 durch Deletion der tPA-Nucleotidsequenz zwischen den Positionen 301 bis 726 (Pennica) gewonnen wird.

8. Verfahren nach Anspruch 6,
**dadurch gekennzeichnet,**
daß man Pseudomonas putida (DSM 2106) züchtet, welches die Plasmide pePa 107.8 oder pePa 143 allein oder zusammen mit Plasmid pePa 119 (DSM 3691 P) enthält, wobei pePa 107.8 gemäß Anspruch 7 definiert ist und das Plasmid pePa 143 ein zur Expression in E.coli und Pseudomonas putida geeigneter tPA-Mutein-Expressionsvektor ist, der durch Ligation von pREM3061 (DSM 3692 P) mit einem ca. 1,45 kb großen Fragment aus pePa 129, das die tPA-Mutein-Nucleotidsequenz gemäß Fig. 1 enthält, gewonnen wird.

9. Plasmid pePa 100.1 gemäß Anspruch 7.

10. Plasmid pePa 107.8 gemäß Anspruch 7.

11. Plasmid pePa 133 gemäß Anspruch 7.

12. Plasmid pePa 137 gemäß Anspruch 7.

13. Plasmid pePa 143 gemäß Anspruch 8.

## Patentansprüche (Patentansprüche für folgende(n) Vertragsstaat(en): AT)

1. Verfahren zur Herstellung eines Plasminogenaktivators durch Expression einer diesen Aktivator codierenden c-DNA, welche in einem für die Wirtszelle geeigneten Vektore eingebaut vorliegt, in einer Prokaryontenzelle,
**dadurch gekennzeichnet,**
daß man einen Vektor verwendet, der entweder strikter Kontrolle unterliegt und dessen Replikation somit von der Replikation des Chromosoms der Wirtszelle nicht oder nur vorübergehend entkoppelbar ist und die Züchtung unter Bedingungen durchführt, bei denen keine relaxierte Kontrolle auftritt, oder einen Promotor verwendet, unter dessen Regulation auch bei entkoppelten Plasmiden nur eine geringe Transkription des Plasminogen-Aktivatorgens erfolgt.

2. Verfahren nach Anspruch 1, dadurch gekennzeichnet, daß man einen Vektor verwendet, dessen Amplifikationsfaktor nicht größer als 3 ist.

3. Verfahren nach Anspruch 1 oder 2, dadurch gekennzeichnet, daß man als Vektor pRSF 1010 (ein Derivat von pREM 3061 (DSM 3692 P), bei dem das Kanamycin-Resistenzgen deletiert wurde), pKN 402, pACYC 177 (DSM 3693 P) oder davon abgeleitete Plasmide, die deren Ori tragen, verwendet.

4. Verfahren nach Anspruch 3, dadurch gekennzeichnet, daß man die c-DNA eines Plasminogenaktivators in pKN 402 verpackt, in eine für diesen Vektor geeignete Prokaryonten-Wirtszelle einführt und die Wirtszelle bei einer Temperatur um 30°C züchtet.

5. Verfahren nach Anspruch 3 oder 4, dadurch gekennzeichnet, daß man als Wirtszelle E. coli DSM 3689 verwendet.

6. Verfahren nach Anspruch 3, dadurch gekennzeichnet, daß man bei pRSF 1010 als Wirtszelle Pseudomonas putida DSM 2106 verwendet.

7. Verfahren nach Anspruch 5,
**dadurch gekennzeichnet,**
daß man E.coli DSM 3689 züchtet, welches Plasmid pePa 100.1 oder/und pePa 107.8 oder/und pePa 133 oder pePa 137 enthält,
wobei das Plasmid pePa 100.1 ein tPA-Eypressionsvektor mit temperatursensitiver Replikationssteuerung ist, der durch Ligation des mit einem temperatursensitiven Replikationsursprung versehenen Plasmids pREM 2334 (DSM 3690 P) mit einem 1,85 kb langen, XhoII-Fragment aus pePa 98.1, das für tPA kodiert und den tac-Promotor enthält, gewonnen wird, wobei pePa 98.1 die tPA-Nucleotidsequenz 192-2165 (Pennica) aus dem Plasmid pBT95 (DSM 3611 P) im Polylinker der Plasmids pKK223-3 (DSM 3694 P) enthält, das Plasmid pePa 107.8 ein zur Expression in E.coli und Pseudomonas putida geeigneter tPA-Expressionsvektor ist, der durch Ligation des Plasmids pREM 3061 (DSM 3692 P) mit dem oben beschriebenen 1,85 kb langen XhoII-Fragment aus pePa 98.1, das für tPA kodiert und den tac-Promotor enthält, gewonnen wird,
das Plasmid pePa 133 ein tPA-Expressionsvektor ist, der durch Ligation eines ca. 1,05 kb großen Fragments aus pKK223-3 (DSM 3694 P), das Transkriptionsterminatoren und die 5'-Portion des β-Lactamasegens enthält, mit einem ca. 2,0 kb großen Fragment aus dem Plasmid pACYC177 (DSM 3693 P), das den Replikationsursprung enthält, und dem oben beschriebenen 1,85 kb langen XhoII-Fragment aus pePa 98.1, das für tPA kodiert und den tac-Promotor enthält, gewonnen wird,
und das Plasmid pePa 137 ein tPA-Mutein-Expressionsvektor ist, der durch Ligation eines Fragments aus pACYC 177 (DSM 3693 P), das den Replikationsursprung enthält, mit einem Fragment aus pePa 129, das die tPA-Mutein-Nucleotidsequenz gemäß Fig. 1 enthält, gewonnen wird, wobei pePa 129.1 aus pePa 98.1 durch Deletion der tPA-Nucleotidsequenz zwischen den Positionen 301 bis 726 (Pennica) gewonnen wird.

8. Verfahren nach Anspruch 6,
**dadurch gekennzeichnet,**
daß man Pseudomonas putida (DSM 2106) züchtet, welches die Plasmide pePa 107.8 oder pePa 143 allein oder zusammen mit Plasmid pePa 119 (DSM 3691 P) enthält, wobei pePa 107.8 gemäß Anspruch 7 definiert ist und das Plasmid pePa 143 ein zur Expression in E.coli und Pseudomonas putida geeigneter tPA-Mutein-Expressionsvektor ist, der durch Ligation von pREM3061 (DSM 3692 P) mit einem ca. 1,45 kb großen Fragment aus pePa 129, das die tPA-Mutein-Nucleotidsequenz gemäß Fig. 1 enthält, gewonnen wird.

## Claims (Claims for the following Contracting State(s): BE, CH, DE, ES, FR, GB, GR, IT, LI, LU, NL, SE)

1. Process for the production of a plasminogen activator by expression of a cDNA, which codes this activator, which is present incorporated in a vector suitable for the host cell, in a prokaryote cell, characterised in that one uses a vector which is subject to strict control and the replication of which cannot be decoupled or only temporarily decoupled from the replication of the chromosome of the host cell and the culturing is carried out under conditions under which no relaxed control occurs or uses a promotor under the regulation of which, also in the case of decoupled plasmids, only a small transcription of the plasminogen activator gene takes place.

2. Process according to claim 1, characterised in that one uses a vector the amplification factor of which is not greater than 3.

3. Process according to claim 1 or 2, characterised in that, as vector, one uses pRSF 1010 (a derivative of pREM 3061 (DSM 3692P), in which the kanamycin-resistant gene has been deleted), pKN 402, pACYC 177 (DSM 3693P) or plasmids derived therefrom which carry their ori.

4. Process according to claim 4, characterised in that one packs the cDNA of a plasminogen activator into pKN 402, introduces into a prokaryote host cell suitable for this vector and cultures the host cell at a temperature of 30°C.

5. Process according to claim 3 or 4, characterised in that one uses E. coli DSM 3689 as host cell.

6. Process according to claim 3, characterised in that, in the case of pRSF 1010, one uses Pseudomonas putida DSM 2106 as host cell.

7. Process according to claim 5, characterised in that one cultures E. coli DSM 2689 which contains plasmid pePa 100.1 and/or pePa 107.8 and/or pePa 133 or pePa 137, whereby the plasmid pePa 100.1 is a tPA expression vector with temperature-sensitive replication control which is obtained by ligation of the plasmid pREM 2334 (DSM 3690 P), provided with a temperature-sensitive replication origin, with a 1.85 kb long Xho II fragment from pePa 98.1 which codes for tPA and contains the tac promotor, whereby pePa 98.1 contains the tPA nucleotide sequence 192 - 2165 (Pennica) from the plasmid pBT95 (DSM 3611 P) in the polylinker of the plasmid pKK 223-3 (DSM 3694 P), the plasmid pePa 107.8 is a tPA expression vector suitable for expression in E. coli and Pseudomonas putida, which is obtained by ligation of the plasmid pREM 3061 (DSM 3692 P) with the above-described 1.85 kb long Xho II fragment from pePa 98.1 which codes for tPA and contains the tac promotor, the plasmid pePa 133 is a tPA expression vector, which is obtained by ligation of an approximately 1.05 kb-sized fragment from pKK223-3 (DSM 3694 P), which contains transcription terminators and the 5'-portion of the β-lactamase gene, with an approximately 2.0 kb-sized fragment from the plasmid pACYC 177 (DSM 3693 P), which contains the replication origin and the above-described 1.85 kb long Xho II fragment from pePa 98.1 which codes for tPA and contains the tac promotor, and the plasmid pePa 137 is a tPA mutein expression vector, which is obtained by ligation of a fragment from pACYC 177 (DSM 3693 P), which contains the replication origin, with a fragment from pePa 129, which contains the tPA mutein nucleotide sequence according to Fig. 1, whereby pePa 129.1 is obtained from pePa 98.1 by deletion of the tPA nucleotide sequence between the positions 301 and 726 (Pennica).

8. Process according to claim 6, characterised in that one cultures Pseudomonas putida (DSM 2106) which contains the plasmids pePa 107.8 or pePa 143 alone or together with plasmid pePa 119 (DSM 3691 P), whereby pePa 107.8 is defined according to claim 7 and the plasmid pePa 143 is a tPA mutein expression vector suitable for the expression in E. coli and Pseudomonas putida which is obtained by ligation of pREM 3061 (DSM 3692 P) with an approximately 1.45 kb sized fragment from pePa 129 which contains the tPA mutein nucleotide sequence according to Fig. 1.

9. Plasmid pePa 100.1 according to claim 7.

10. Plasmid pePa 107.8 according to claim 7.

11. Plasmid pePa 133 according to claim 7.

12. Plasmid pePa 137 according to claim 7.

13. Plasmid pePa 143 according to claim 8.

## Claims (Claims for the following Contracting State(s): AT)

1. Process for the production of a plasminogen activator by expression of a cDNA, which codes this activator, which is present incorporated in a vector suitable for the host cell, in a prokaryote cell, characterised in that one uses a vector which is subject to strict control and the replication of which cannot be decoupled or only temporarily decoupled from the replication of the chromosome of the host cell and the culturing is carried out under conditions under which no relaxed control occurs or uses a promotor under the regulation of which, also in the case of decoupled plasmids, only a small transcription of the plasminogen activator gene takes place.

2. Process according to claim 1, characterised in that one uses a vector the amplification factor of which is not greater than 3.

3. Process according to claim 1 or 2, characterised in that, as vector, one uses pRSF 1010 (a derivative of pREM 3061 (DSM 3692P), in which the kanamycin-resistant gene has been deleted), pKN 402, pACYC 177 (DSM 3693P) or plasmids derived therefrom which carry their ori.

4. Process according to claim 4, characterised in that one packs the cDNA of a plasminogen activator into pKN 402, introduces into a prokaryote host cell suitable for this vector and cultures the host cell at a temperature of 30°C.

5. Process according to claim 3 or 4, characterised in that one uses E. coli DSM 3689 as host cell.

6. Process according to claim 3, characterised in that, in the case of pRSF 1010, one uses Pseudomonas putida DSM 2106 as host cell.

7. Process according to claim 5, characterised in that one cultures E. coli DSM 2689 which contains plasmid pePa 100.1 and/or pePa 107.8 and/or pePa 133 or pePa 137, whereby the plasmid pePa 100.1 is a tPA expression vector with temperature-sensitive replication control which is obtained by ligation of the plasmid pREM 2334 (DSM 3690 P), provided with a temperature-sensitive replication origin, with a 1.85 kb long Xho II fragment from pePa 98.1 which codes for tPA and contains the tac promotor, whereby pePa 98.1 contains the tPA nucleotide sequence 192 - 2165 (Pennica) from the plasmid pBT95 (DSM 3611 P) in the polylinker of the plasmid pKK 223-3 (DSM 3694 P), the plasmid pePa 107.8 is a tPA expression vector suitable for expression in E. coli and Pseudomonas putida, which is obtained by ligation of the plasmid pREM 3061 (DSM 3692 P) with the above-described 1.85 kb long Xho II fragment from pePa 98.1 which codes for tPA and contains the tac promotor, the plasmid pePa 133 is a tPA expression vector, which is obtained by ligation of an approximately 1.05 kb-sized fragment from pKK 223-3 (DSM 3694 P), which contains transcription terminators and the 5'-portion of the β-lactamase gene, with an approximately 2.0 kb-sized fragment from the plasmid pACYC 177 (DSM 3693 P), which contains the replication origin and the above-described 1.85 kb long Xho II fragment from pePa 98.1 which codes for tPA and contains the tac promotor, and the plasmid pePa 137 is a tPA mutein expression vector, which is obtained by ligation of a fragment from pACYC 177 (DSM 3693 P), which contains the replication origin, with a fragment from pePa 129, which contains the tPA mutein nucleotide sequence according to Fig. 1, whereby pePa 129.1 is obtained from pePa 98.1 by deletion of the tPA nucleotide sequence between the positions 301 and 726 (Pennica).

8. Process according to claim 6, characterised in that one cultures Pseudomonas putida (DSM 2106) which contains the plasmids pePa 107.8 or pePa 143 alone or together with plasmid pePa 119 (DSM 3691 P), whereby pePa 107.8 is defined according to claim 7 and the plasmid pePa 143 is a tPA mutein expression vector suitable for the expression in E. coli and Pseudomonas putida which is obtained by ligation of pREM 3061 (DSM 3692 P) with an approximately 1.45 kb sized fragment from pePa 129 which contains the tPA mutein nucleotide sequence according to Fig. 1.

## Revendications (Revendications pour l'(les) Etat(s) contractant(s) suivant(s): BE, CH, DE, ES, FR, GB, GR, IT, LI, LU, NL, SE)

1. Procédé pour la préparation d'un activateur du plasminogène par expression dans une cellule procaryote d'un ADNc codant cet activateur, qui est incorporé dans un vecteur convenant pour la cellule hôte, caractérisé en ce que l'on utilise un vecteur qui subit un contrôle strict et dont la réplication ne peut donc pas être découplée de la réplication du chromosome de la cellule hôte ou ne peut être découplée que provisoirement et la culture est réalisée dans des conditions dans lesquelles il ne se produit aucun contrôle relâché, ou l'on utilise un promoteur sous la régulation duquel il ne se produit qu'une faible transcription du gène de l'activateur du plasminogène même dans le cas de plasmides découplés.

2. Procédé selon la revendication 1, caractérisé en ce que l'on utilise un vecteur dont le facteur d'amplification n'est pas supérieur à 3.

3. Procédé selon la revendication 1 ou 2, caractérisé en ce que l'on utilise comme vecteur pRSF 1010 (DSM 3693 P) (un dérivé de pREM 3061 (DSM 3692 P) dans lequel le gène de résistance à la kanamycine a été supprimé par délétion), pKN 402, pACYC 177 ou des plasmides dérivés de ceux-ci qui portent leur ori.

4. Procédé selon la revendication 3, caractérisé en ce que l'on empaquette l'ADNc d'un activateur du plasminogène dans pKN 402, on l'introduit dans une cellule hôte procaryote convenant à ce vecteur et on cultive la cellule hôte à une température voisine de 30°C.

5. Procédé selon la revendication 3 ou 4, caractérisé en ce que l'on utilise comme cellule hôte E. coli DSM 3689.

6. Procédé selon la revendication 3, caractérisé en ce que, dans le cas de pRSF 1010, on utilise comme cellule hôte Pseudomonas putida DSM 2106.

7. Procédé selon la revendication 5, caractérisé en ce que l'on cultive E. coli DSM 3689, qui contient le plasmide pePa 100.1 et/ou pePa 107.8 et/ou pePa 133 ou pePa 137, le plasmide pePa 100.1 étant un vecteur d'expression du t-PA à commande de réplication sensible à la température qui est obtenu par ligature du plasmide pREM 2334 (DSM 3690) muni d'une origine de réplication sensible à la température avec un fragment Xho II de 1,85 kb provenant de pePa 98.1 qui code pour le t-PA et qui contient le promoteur tac, pePa 98.1 contenant la séquence nucléotidique du t-PA 192-2165 (Pennica) provenant du plasmide pBT95 (DSM 3611 P) dans le polylieur du plasmide pKK223-3 (DSM 3694 P), le plasmide pePa 107.8 étant un vecteur d'expression du t-PA approprié pour l'expression dans E. coli et Pseudomonas putida, qui est obtenu par ligature du plasmide pREM 3061 (DSM 3692 P) avec le fragment Xho II de 1,85 kb provenant de pePa 98.1 décrit ci-dessus qui code pour le t-PA et qui contient le promoteur tac, le plasmide pePa 133 étant un vecteur d'expression du t-PA qui est obtenu par ligature d'un fragment d'environ 1,05 kb provenant de pKK223-3 (DSM 3694 P), qui contient des terminateurs de transcription et la partie 5' du gène de la β-lactamase, avec un fragment d'environ 2,0 kb provenant du plasmide pACYC177 (DSM 3693 P) qui contient l'origine de réplication et le fragment XhoII de 1,85 kb provenant de pePa 98.1 décrit ci-dessus, qui code pour le t-PA et qui contient le promoteur tac, et le plasmide pePa 137 étant un vecteur d'expression de mutéine du t-PA qui est obtenu par ligature d'un fragment provenant de pACYC 177 (DSM 3693 P) qui contient l'origine de réplication, avec un fragment provenant de pePa 129 qui contient la séquence nucléotidique de mutéine du t-PA selon la figure 1, pePa 129.1 étant obtenu à partir de pePa 98.1 par délétion de la séquence nucléotidique du t-PA entre les positions 301 et 726 (Pennica).

8. Procédé selon la revendication 6, caractérisé en ce que l'on cultive Pseudomonas putida (DSM 2106) qui contient les plasmides pePa 107.8 ou pePa 143 seuls ou avec le plasmide pePa 119 (DSM 3691 P), pePa 107.8 étant défini selon la revendication 7 et le plasmide pePa 143 étant un vecteur d'expression de la mutéine du t-PA approprié pour l'expression dans E. coli et Pseudomonas putida, qui est obtenu par ligature de pREM3061 (DSM 3692 P) avec un fragment d'environ 1,45 kb provenant de pePa 129 qui contient la séquence nucléotidique de la mutéine du t-PA selon la figure 1.

9. Plasmide pePa 100.1 selon la revendication 7.

10. Plasmide pePa 107.8 selon la revendication 7.

11. Plasmide pePa 133 selon la revendication 7.

12. Plasmide pePa 137 selon la revendication 7.

13. Plasmide pePa 143 selon la revendication 8.

## Revendications (Revendications pour l'(les) Etat(s) contractant(s) suivant(s): AT)

1. Procédé pour la préparation d'un activateur du plasminogène par expression dans une cellule procaryote d'un ADNc codant cet activateur, qui est incorporé dans un vecteur convenant pour la cellule hôte, caractérisé en ce que l'on utilise un vecteur qui subit un contrôle strict et dont la réplication ne peut donc pas être découplée de la réplication du chromosome de la cellule hôte ou ne peut être découplée que provisoirement et la culture est réalisée dans des conditions dans lesquelles il ne se produit aucun contrôle relâché, ou l'on utilise un promoteur sous la régulation duquel il ne se produit qu'une faible transcription du gène de l'activateur du plasminogène même dans le cas de plasmides découplés.

2. Procédé selon la revendication 1, caractérisé en ce que l'on utilise un vecteur dont le facteur d'amplification n'est pas supérieur à 3.

3. Procédé selon la revendication 1 ou 2, caractérisé en ce que l'on utilise comme vecteur pRSF 1010 (DSM 3693 P) (un dérivé de pREM 3061 (DSM 3692 P) dans lequel le gène de résistance à la kanamycine a été supprimé par délétion), pKN 402, pACYC 177 ou des plasmides dérivés de ceux-ci qui portent leur ori.

4. Procédé selon la revendication 3, caractérisé en ce que l'on empaquette l'ADNc d'un activateur du plasminogène dans pKN 402, on l'introduit dans une cellule hôte procaryote convenant à ce vecteur et on cultive la cellule hôte à une température voisine de 30°C.

5. Procédé selon la revendication 3 ou 4, caractérisé en ce que l'on utilise comme cellule hôte E. coli DSM 3689.

6. Procédé selon la revendication 3, caractérisé en ce que, dans le cas de pRSF 1010, on utilise comme cellule hôte Pseudomonas putida DSM 2106.

7. Procédé selon la revendication 5, caractérisé en ce que l'on cultive E. coli DSM 3689, qui contient le plasmide pePa 100.1 et/ou pePa 107.8 et/ou pePa 133 ou pePa 137, le plasmide pePa 100.1 étant un vecteur d'expression du t-PA à commande de réplication sensible à la température qui est obtenu par ligature du plasmide pREM 2334 (DSM 3690) muni d'une origine de réplication sensible à la température avec un fragment Xho II de 1,85 kb provenant de pePa 98.1 qui code pour le t-PA et qui contient le promoteur tac, pePa 98.1 contenant la séquence nucléotidique du t-PA 192-2165 (Pennica) provenant du plasmide pBT95 (DSM 3611 P) dans le polylieur du plasmide pKK223-3 (DSM 3694 P), le plasmide pePa 107.8 étant un vecteur d'expression du t-PA approprié pour l'expression dans E. coli et Pseudomonas putida, qui est obtenu par ligature du plasmide pREM 3061 (DSM 3692 P) avec le fragment Xho II de 1,85 kb provenant de pePa 98.1 décrit ci-dessus qui code pour le t-PA et qui contient le promoteur tac, le plasmide pePa 133 étant un vecteur d'expression du t-PA qui est obtenu par ligature d'un fragment d'environ 1,05 kb provenant de pKK223-3 (DSM 3694 P), qui contient des terminateurs de transcription et la partie 5' du gène de la β-lactamase, avec un fragment d'environ 2,0 kb provenant du plasmide pACYC177 (DSM 3693 P) qui contient l'origine de réplication et le fragment XhoII de 1,85 kb provenant de pePa 98.1 décrit ci-dessus, qui code pour le t-PA et qui contient le promoteur tac, et le plasmide pePa 137 étant un vecteur d'expression de mutéine du t-PA qui est obtenu par ligature d'un fragment provenant de pACYC 177 (DSM 3693 P) qui contient l'origine de réplication, avec un fragment provenant de pePa 129 qui contient la séquence nucléotidique de mutéine du t-PA selon la figure 1, pePa 129.1 étant obtenu à partir de pePa 98.1 par délétion de la séquence nucléotidique du t-PA entre les positions 301 et 726 (Pennica).

8. Procédé selon la revendication 6, caractérisé en ce que l'on cultive Pseudomonas putida (DSM 2106) qui contient les plasmides pePa 107.8 ou pePa 143 seuls ou avec le plasmide pePa 119 (DSM 3691 P), pePa 107.8 étant défini selon la revendication 7 et le plasmide pePa 143 étant un vecteur d'expression de la mutéine du t-PA approprié pour l'expression dans E. coli et Pseudomonas putida, qui est obtenu par ligature de pREM3061 (DSM 3692 P) avec un fragment d'environ 1,45 kb provenant de pePa 129 qui contient la séquence nucléotidique de la mutéine du t-PA selon la figure 1.
